(19) Europäisches Patentamt
European Patent Office
Office européen des brevets

(11) EP 3 539 468 A1

(12) EUROPEAN PATENT APPLICATION

(43) Date of publication:
18.09.2019 Bulletin 2019/38

(51) Int Cl.:
A61B 5/053 (2006.01)    A61B 5/00 (2006.01)

(21) Application number: 19161966.7

(22) Date of filing: 11.03.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 12.03.2018 EP 18161245

(71) Applicant: Stichting IMEC Nederland
5656 AE  Eindhoven (NL)

(72) Inventor: LEE, Seulki
3001 Leuven (BE)

(74) Representative: AWA Sweden AB
P.O. Box 45086
104 30 Stockholm (SE)

(54) **A DEVICE AND A METHOD FOR BIOIMPEDANCE MEASUREMENT**

(57)    A device for bioimpedance measurement comprises: a carrier (102), which is configured to be worn by a person, a plurality of electrodes (120), which are arranged at a distance from a surface of the carrier (102) facing the person when the carrier (102) is worn, the plurality of electrodes (120) being configured for forming a capacitive coupling to the person when the carrier (102) is worn, a bioimpedance measurement unit (110), which is supported by the carrier (102) and which is configured to, via at least one pair of electrodes among said plurality of electrodes (120), provide a stimulation signal and acquire a measurement signal indicative of bioimpedance of the person, wherein the bioimpedance measurement unit (110) is configured to take a distance between the at least one pair of electrodes and skin of the person into account in order to estimate a static physiological characteristic of a bioimpedance.

Fig. 1

EP 3 539 468 A1

**Description**

Technical field

The present inventive concept relates to a device and a method for bioimpedance measurement.

Background

Bioimpedance signals are of increasing interest to use for various purposes. Bioimpedance signals may be used in monitoring of health of a person, but may also be used in determining characteristic information of the person.
Bioimpedance measurements may be performed with relatively simple equipment causing minimal or at least low inconvenience to the person on which the bioimpedance measurements are performed. This implies that the relatively cheap equipment for performing bioimpedance measurements, which does not significantly affect the person wearing equipment, forms an interesting alternative for being worn over long time-frames and provide.
In many uses of bioimpedance measurements, a change is being measured in order to sense a changing signal, such as using the bioimpedance measurement to detect hand gestures or using the bioimpedance measurement for detecting a modulation of a measurement signal by a cardiac or pulmonary activity.
However, if it is desired to detect a static physiological characteristic, such as an absolute value and/or phase of the bioimpedance of the person, there is a need for an accurate control of a relationship between electrodes of the bioimpedance equipment and skin of the person. This may cause inconvenience to the person wearing the equipment, especially if the equipment is to be worn for a long period of time, since the equipment may need to be worn with a tight fit on the person, such that a good contact between the electrodes and the skin is ensured.
Therefore, it would be desired to improve bioimpedance measurement equipment so as to improve convenience of the person wearing the equipment.

Summary

An objective of the present inventive concept is to improve convenience of use of a device for bioimpedance measurements, while enabling determining of a static physiological characteristic of the bioimpedance.
This and other objects of the present inventive concept are at least partly met by the invention as defined in the independent claims. Preferred embodiments are set out in the dependent claims.
According to a first aspect, there is provided a device for bioimpedance measurement, said device comprising: a carrier, which is configured to be worn by a person, a plurality of electrodes, which are arranged at a distance from a surface of the carrier facing the person when the carrier is worn, the plurality of electrodes being configured for forming a capacitive coupling to the person when the carrier is worn, a bioimpedance measurement unit, which is supported by the carrier and which is configured to, via at least one pair of electrodes among said plurality of electrodes, provide a stimulation signal and acquire a measurement signal indicative of bioimpedance of the person, wherein the bioimpedance measurement unit is configured to take a distance between the at least one pair of electrodes and skin of the person into account in order to estimate a static physiological characteristic of a bioimpedance.
It is an insight of the present inventive concept that electrodes may be arranged in a non-contact relation to the skin of the person. The electrodes will thus form a capacitive coupling the person when the carrier is worn.
Thanks to having a non-contact relation between the electrodes and the skin of the person, the carrier need not necessarily be arranged in a tight relationship to a body part on which the carrier is worn. For instance, a wristband need not be arranged tightly around the wrist of the person. This may significantly improve convenience to the person wearing the device and make the device more suitable for long-term use.
Further, the non-contact relation between the electrodes and the skin of the person also improves safety of using the device. A device having electrodes directly coupled to skin of the person may need a safety mechanism to prevent DC current to be provided into the body, e.g. in case of device failure. However, with a non-contact relation between electrodes and skin of the person, there is an insulating interface between the electrodes and skin, which implies that a separate safety mechanism may not be needed.
On the other hand, the capacitive coupling between the electrodes and the skin may imply that there is a very large impedance caused by the capacitive coupling, as there is an insulator between the electrodes and the skin. This implies that it would be very difficult to determine a static physiological characteristic of bioimpedance in a body portion, such as a skin portion, of the person. The bioimpedance may be very small in relation to the impedance caused by the capacitive coupling between the electrodes and the skin.
However, according to the present inventive concept, a bioimpedance measurement unit is configured to take a distance between the electrodes and the skin into account in order to determine a static physiological characteristic, such as an absolute value and/or phase of the bioimpedance. Thus, the bioimpedance measurement unit may be configured to have access to information of the distance between the electrodes and the skin. The bioimpedance measurement unit may thus use the information so as to determine the bioimpedance even though the bioimpedance measurement involves a large impedance caused by a capacitive coupling between the electrodes and the skin.

There may be different ways for the bioimpedance measurement unit to know the distance between the electrodes and the skin such that the bioimpedance measurement unit may have access to the information of distance and, hence, may take the distance into account when determining an absolute value and/or phase of the bioimpedance. For instance, the device may be configured in such manner that the distance to the skin of the person will be set by a way in which the carrier is worn by the person. The carrier may be arranged with an at least relatively tight fit between the carrier and the skin of the person. Then, a distance between an electrode and a surface of the carrier in contact with the skin is known and may be used by the bioimpedance measurement unit as a distance between the electrode and the skin. Alternatively, as will be explained in more detail below, the device may comprise a distance detector for determining the distance between the electrode and the skin.

The bioimpedance measurement unit may take the distance between the electrodes and the skin into account by estimating an impedance of the capacitive coupling between the electrodes and skin as a function of the distance. This implies that the bioimpedance measurement unit may quantitatively determine an impact of the capacitive coupling between the electrodes and skin on the measurement signal, such that the impact may be compensated for and that an absolute value and/or phase of the bioimpedance may be determined. It should be realized that several different ways of processing the measurement signal may be contemplated for determining the absolute value and/or phase of the bioimpedance.

The bioimpedance measurement unit may take the distance between the electrodes and the skin into account by using pre-determined information on how the measurement signal is affected by different distances. Thus, a calibration may be performed before the device is taken into use, e.g. an individual calibration for the person who is going to wear the device, wherein the calibration may be performed for different distances between the electrodes and the skin such that appropriate calibration data may be used in dependence of the distance between the electrodes and the skin in acquiring of a particular measurement signal.

The term "static physiological characteristic" should be construed as a characteristic bioimpedance of the person that does not change with any activity of the person, such as movements of the person or activity of an organ, such as heart or lungs, of the person. It is realized by the inventors that bioimpedance measurements on corresponding body parts (such as a wrist, an ankle, a chest portion) of different people typically yield characteristic and repeatable results. Hence, the bioimpedance of the person in a particular portion of a body part may provide a static characteristic, which may differ from other persons. The static physiological characteristic may be determined as an absolute value and/or phase of the bioimpedance of the person. However, with a well-defined set-up of the bioimpedance measurement, for a specific distance between the electrodes and the skin, a characteristic impedance measurement as a combination of the capacitive coupling between electrodes and skin and the bioimpedance may be determined. Thus, it may not be necessary to actually extract the contribution from the bioimpedance, but rather the combined impedance of the capacitive coupling and the bioimpedance may be used as the static physiological characteristic in dependence on a distance between the electrodes and the skin. A measured combined impedance may thus be compared with calibration data in order to determine whether the measured combined impedance forming a representation of the static physiological characteristic corresponds to the calibration data and, hence, whether the measurement is performed on the person for which calibration data was acquired.

The carrier may be configured to be worn on a body part of the person. Advantageous examples of body parts include a wrist, an ankle, an arm portion, a leg portion or a chest portion of the person.

According to an embodiment, the carrier may be in the form of a strap adapted to be worn about a body part of the user. The strap may advantageously be a wrist band, an ankle strap, an arm strap, a leg strap or a chest strap.

The carrier may be formed of, or comprise at least between the electrodes and a surface of the carrier which is to face skin of the person when being worn, an insulating material. The insulating material may be selected from a material which has well-known and homogeneous insulating properties, such that impact of the carrier material on the capacitive coupling between the electrodes and the skin may be predictable.

According to an embodiment, the carrier may include a flexible material, such as a woven material, a textile, a rubber or silicone material, which may facilitate the carrier being worn by the person. The strap may alternatively include a (more) rigid material. The strap may for example include leather or a plastic material.

The electrodes may be embedded in the carrier in order to ensure that there is an insulating material between the electrodes and the skin of the person when the carrier is worn. However, if the carrier has a well-defined surface which is to face the skin of the person, the carrier may also have an opposite surface to the surface facing the skin and the electrodes may in such case not necessarily be embedded in the carrier. Rather, the electrodes may be arranged on the opposite surface, which will not be in contact with skin of the person.

The bioimpedance measurement unit may comprise a bioimpedance measurement circuit, which may be any circuitry being configured to measure an impedance in portion(s) of the person via at least one pair of electrodes.

The bioimpedance measurement circuit may be configured to perform bioimpedance measurements via groups of electrodes including four electrodes in two pairs of electrodes. A measurement via four electrodes may be referred to as a tetrapolar measurement. Such a configuration may enable reliable and accurate impedance measurements.

The bioimpedance measurement circuit may alternatively be configured to perform bioimpedance measurements via groups of electrodes including only a single pair of electrodes. A measurement via two electrodes may be referred to as a bipolar measurement. Such a configuration enables impedance measurements to be performed in a plurality of different portions of the person via a smaller number of electrodes compared to a bioimpedance measurement circuit configured for tetrapolar measurements.

The plurality of electrodes may be any type of electrodes suitable for electrical measurements, in particular impedance measurements, on the person.

The plurality of electrodes may be distributed along the carrier such that the electrodes are distributed over an area of the body part on which the carrier is adapted to be worn. This may facilitate measuring bioimpedance in a plurality of positions on the body part of the person.

However, it should be realized that the device may be configured only for detection of bioimpedance in a single position. Thus, the device may comprise only two electrodes (for a bipolar measurement) or four electrodes (for a tetrapolar measurement).

According to an embodiment, the device further comprises a distance detector, which is supported by the carrier, and which is configured to determine a distance between at least one pair of electrodes and the skin of the person.

Thanks to the device comprising a distance detector, a distance between the electrodes and skin may be determined by the device. This implies that the distance may change between bioimpedance measurements and such change of distance may be taken into account thanks to the distance detector being able to provide information of the distance applying to a particular measurement.

The distance detector may be arranged on a surface of the carrier which is configured to face the skin of the person when the carrier is worn. Thus, the distance detector may be configured to determine a distance between a surface of the carrier and the skin of the person. An actual distance between the electrodes and the skin may then be determined as the distance between the surface of the carrier and the skin, as determined by the distance detector, and a constant distance between a position in the carrier in which the electrode is arranged and the surface of the carrier.

Hence, it should be understood that the distance detector may not necessarily directly determine the distance between the electrodes and the skin, but may be configured to detect a (partial) distance which may allow the distance between the electrodes and the skin to be determined.

According to an embodiment, the device may comprise a plurality of distance detectors. Each distance detector may be associated with one or more pairs of electrodes in the carrier. Thus, the plurality of distance detectors may determine a plurality of distances between electrodes and the skin, such that a distance applying to a particular pair of electrodes may be determined. This may allow correct determination of the bioimpedance, even if the carrier is not arranged with a consistent distance to the skin for the entire carrier. For instance, if the carrier is worn as a strap around a body part, the carrier may not have a single common distance to the skin throughout a circumference of the carrier.

It may not be necessary for the device to comprise one distance detector for each pair of electrodes in the carrier. However, a number of distance detectors may be distributed along the carrier. For instance, a single distance detector may be shared by two pairs, four pairs, or eight pairs of electrodes for indicating the distance between the electrodes and skin.

According to an embodiment, the distance detector is configured to detect a distance based on an optical measurement or an electrical measurement.

Thus, the distance detector may use a relatively simple technology for determining a distance between the electrodes and the skin. The distance detector may be configured to detect a property which is dependent on a distance.

According to an embodiment, the distance detector may be configured to detect a time of propagation of a signal or an attenuation of a signal. This may be used in an optical or electrical measurement, wherein an electromagnetic wave, such as a light wave or radio frequency wave, is transmitted and a reflected wave is detected. For instance, the distance detector may include a light source configured to transmit a light pulse towards the skin and a light detector for detecting light being reflected by the skin.

According to another embodiment, the distance detector may be configured to detect an electrical property that may be dependent on the distance between the electrodes and the skin. For instance, the distance detector may comprise a circuit configured to detect an impedance caused by the distance between the electrodes and the skin. Thus, the device may comprise bioimpedance measurement electrodes for determining the bioimpedance of the person and distance measurement electrodes for determining the impedance between the carrier (bioimpedance measurement electrodes) and the skin.

In an embodiment, the same electrodes may be used both for determining the bioimpedance of the person and for detecting a distance between the electrodes and the skin. Electrodes may shift between being used as bioimpedance measurement electrodes or being used as distance measurement electrodes. Thus, in a first measurement, at least a first pair of electrodes may be used as bioimpedance measurement electrodes while at least a second pair of electrodes may be used as distance measurement electrodes. In a second measurement, the at least a second pair of electrodes may be used as bioimpedance measurement electrodes while the at least a first pair of electrodes may be used as distance measurement electrodes.

According to yet another embodiment, the same electrodes may be used for detecting a distance between the electrodes and the skin and for determining the bioimpedance of the person within the same measurement. For instance, the electrodes may first be used for detecting the distance and may then be used for determining the bioimpedance of the person. In fact, the distance may be measured using a different frequency than used in determining the bioimpedance of the person, such that the same electrodes may be used at the same time for determining the bioimpedance of the person and for detecting the distance between the electrodes and the skin.

According to an embodiment, the bioimpedance measurement unit is configured to estimate a contribution to a measured signal from an insulating interface formed by the distance between the electrodes and the skin of the person in order to determine an absolute value and/or phase of an bioimpedance.

This implies that the bioimpedance measurement unit may estimate an impedance caused by a thickness of an insulating material (carrier material and airgap between carrier and skin). Thus, an estimated impedance of the capacitive coupling between the electrodes and the skin may be determined, which may allow determining an absolute value and/or phase of the bioimpedance from the measurement signal.

According to an embodiment, the bioimpedance measurement unit comprises a bioimpedance measurement circuit, which is configured to perform bioimpedance measurements on the person via a plurality of different groups among said plurality of electrodes.

This implies that the bioimpedance measurement circuit may be configured to determine bioimpedance in a plurality of positions of the person in relation to the carrier. For instance, the bioimpedance measurement circuit may be configured to determine the bioimpedance in a plurality of different positions along a circumference of a wrist of the person.

The determining of the bioimpedance in a plurality of positions of the person may be used e.g. in identification of a person, since it is realized by the inventors that bioimpedance measurements on corresponding body parts (such as a wrist, an ankle, a chest portion) of different people typically yield characteristic and repeatable results.

The bioimpedance measurement circuit may be configured to control the performing of bioimpedance measurements, such that the circuit may be configured to acquire measurement signals for a plurality of different groups of electrodes. This implies that the bioimpedance measurement circuit may be re-used for acquiring measurement signals from a plurality of different groups.

According to an embodiment, the bioimpedance measurement unit comprises a bias network, which is configured to provide an operating voltage at an input terminal of the bioimpedance measurement circuit.

The bioimpedance measurement unit may be configured to induce a voltage in the skin of the person via a capacitive coupling and may also be configured to receive a measurement signal via a capacitive coupling. This implies that a detected AC measurement signal is not detected in relation to a defined DC baseline. The bias network may therefore be used for setting a fixed operating point at an input terminal of a bioimpedance measurement circuit.

The use of a bias network may thus facilitate correct processing of a received signal by the bioimpedance measurement circuit. Also, the bias network may ensure that the measurement signal is provided in such manner that an identical bioimpedance measurement circuit used for processing a measurement signal acquired with skin electrodes in contact with skin may also be used for processing the measurement signal acquired with the capacitively coupled electrodes according to the present disclosure.

According to an embodiment, the bioimpedance measurement unit comprises a signal generator, which is configured to generate an AC stimulation current signal without a DC offset component of the current signal.

The bioimpedance measurement unit may comprise a signal generator for generating the AC stimulation current signal. The AC stimulation current signal may be provided to a pair of electrodes in order to induce a varying voltage in the skin of the person via the capacitive coupling between the electrodes and the skin.

Since the electrodes are capacitively coupled to the skin, there is no or insignificant risk that a DC current will be induced in the person via the (insulating) interface between the electrodes and the skin. Therefore, the bioimpedance measurement unit does not need to include a capacitor between the signal generator and the electrodes in order to remove any DC component.

However, the induced AC signal in the skin of the person via the stimulating signal and the detected AC measurement signal do not have a defined DC baseline in the person. Therefore, it may be desired to set a fixed operating point at the electrode terminals both for the stimulating signal and for acquiring the measurement signal. In this sense, it is advantageous to generate an AC stimulation current signal without a DC offset component of the current signal, such that an operating point may be accurately set.

Thus, by the signal generator generating an AC stimulation current signal without a DC offset component of the current signal, a desired operating point of the bioimpedance measurement circuit may be easily set.

The signal generator may generate the AC stimulation current signal by directly generating an AC signal without a DC offset component. According to an alternative, an AC stimulation current signal may be generated and may then be received by a DC eliminating unit which may remove any DC offset component from the AC stimulation current signal.

Further, the bias network may be connected at both electrode terminals for receiving the stimulating signal to induce a varying voltage in the skin of the person and electrode terminals for acquiring the measurement signal. The bias network may in this manner control the operating point of all electrode terminals.

According to an embodiment, the bioimpedance measurement unit is configured to filter the measurement signal in time domain to eliminate large changes in magnitude of the measurement signal.

The capacitive coupling between the electrodes and skin introduces a large impedance in a set-up for acquiring bioimpedance measurements. Thus, a change in the distance between the electrodes and skin may cause a large change in the impedance measurement, which may be far larger than a normal variation of the measured AC signal. Therefore, when a large change in magnitude of the measurement signal occurs, it may be concluded that the change is due to a change in the distance between the electrodes and skin.

By means of eliminating the large changes in magnitude from the measurement signal, distance changes may not be allowed to affect determination of the bioimpedance.

The filter may eliminate a time period from the measurement signal associated with the large change in magnitude.

When a large change in magnitude of the measurement signal is detected, an indication may be provided that there is a need for updating a distance between the electrodes and skin. Thus, a new distance measurement may be performed using the distance detector in order to determine the distance between the electrodes and skin after the change, such that the updated distance may be used in processing of the measurement signal.

According to an embodiment, the device further comprises a memory storing calibration data, wherein the bioimpedance measurement unit is configured to process the measurement signal using the calibration data.

This implies that the device may be calibrated for enabling taking a distance between at least one pair of electrodes and skin of the person into account in order to estimate the static physiological characteristic of a bioimpedance. For instance, the measurement signal may be directly compared to calibration data in order to determine whether the measurement signal corresponds to the calibration data so as to enable determination whether the measurement signal is acquired for the same person, for whom calibration data was acquired. Thus, an effect of the capacitive coupling may not need to be directly compensated for in the measurement signal, but may be handled through the calibration data instead in order to use the static physiological characteristic of a person for identification of the person.

According to an embodiment, the calibration data comprises bioimpedance measurement results for different distances between at least one pair of electrodes and the skin of the person.

The calibration data may allow comparing the measurement signal to different data sets in the calibration data depending on the distance between the electrodes and skin. Thus, the measurement signal may be compared to a corresponding data set in the calibration data based on the distance between the electrodes and skin.

Further, the calibration data may be acquired for the specific person who is going to wear the device. This implies that the calibration data may be individualized to be specific to the person wearing the device and to different distances between the electrodes and skin.

According to an embodiment, the carrier is in the form of a wristband.

This may be advantageous in allowing the person to wear the device without causing inconvenience to the person, as people are normally used to wearing wristbands (such as watches, bracelets). Further, the device may be integrated in a more complex device provided in the form of a wristband, such as a smart watch or smart bracelet.

According to an embodiment, the bioimpedance measurement unit is configured to determine a measurement data set based on measurement signals acquired via a plurality of different groups of electrodes and based on each frequency channel of a set of frequency channels of the stimulation signal, wherein the device further comprises an identification unit, which is configured to process the measurement data set in relation to at least one reference data set for identifying the person.

By means of determining the bioimpedance in a plurality of positions of the person (via a plurality of different groups of electrodes) and for a plurality of frequencies, a measurement data set may be acquired which may allow reliably identifying a person or confirming an identity of the person.

The device may thus also comprise an identification unit which may be configured to process measurement data sets in relation to at least one reference data set in order to identify the person. This implies that the device may provide a reliable identification of the person, which may be used in various applications.

Hence, by wearing the device, the person may always carry an identification equipment which may be used for authenticating the person, e.g. for providing access to a building or a computer network, or for making money transactions. This implies that the device may form a suitable equipment for authentication, which may remove needs of passwords or more advanced equipment to be installed at a point where authentication is needed.

The identification unit may be configured to process the measurement data set in relation to a plurality of reference data sets. This may imply that the device may be able to differentiate between a plurality of identities of the person and determine which of the persons is wearing the device.

According to a second aspect, there is provided a method for bioimpedance measurement, said method comprising: providing a stimulation signal; acquiring a measurement signal, wherein the providing of the stimulation signal and the receiving of the measurement signal is performed via at least one pair of electrodes which are capacitively coupled to the person; accessing information of a distance between the at least one pair of electrodes and skin of the person; and processing the measurement signal in view of the distance between the at least one pair of electrodes and the skin of the person in order to estimate a static physiological characteristic of a bioimpedance.

Effects and features of this second aspect are largely analogous to those described above in connection with the first aspect. Embodiments mentioned in relation to the first aspect are largely compatible with the second aspect.

Thanks to the accessing of information of a distance between the at least one pair of electrodes and skin of the person and processing of the measurement signal in view of the distance between the at least one pair of electrodes and the skin of the person, it is possible to determine a static physiological characteristic of an bioimpedance even if using electrodes which are not in contact with skin of the person.

According to an embodiment, said accessing of information of the distance comprises measuring the distance between the at least one pair of electrodes and the skin of the person using a distance detector.

Thus, the distance may be measured in association with acquiring of the measurement signal, such that the distance between the electrodes and skin when acquiring the measurement signal may be known. The measured distance may be stored in a memory or directly provided to a bioimpedance measurement unit such that the bioimpedance measurement unit may have access to the measured distance when processing the measurement signal.

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

Fig. 1 is a schematic view of a device according to an embodiment.
Figs 2-3 are schematic views of different arrangements of a carrier of the device in relation to a body part during bioimpedance measurements.
Fig. 4 is a flow chart illustrating a method for bioimpedance measurement according to an embodiment.

Detailed description

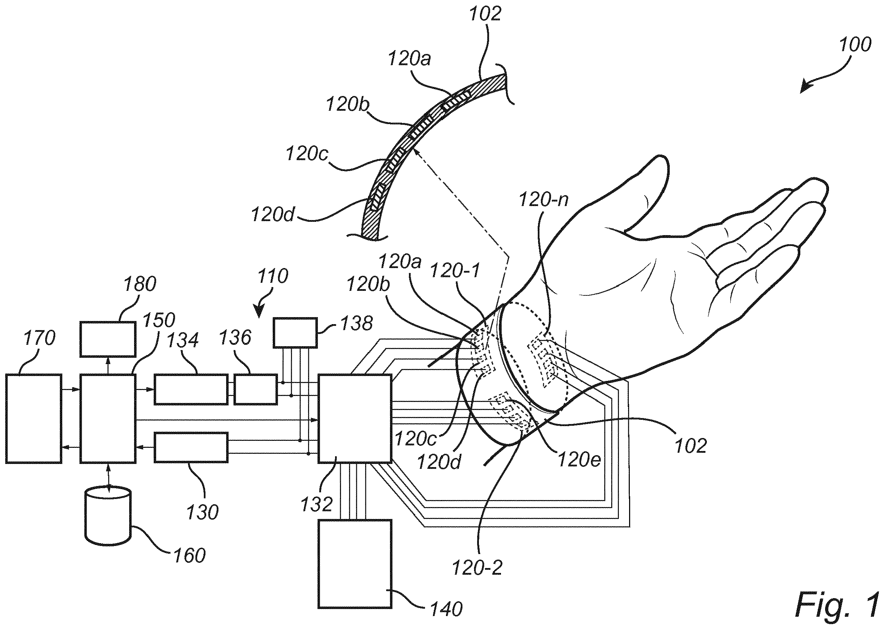

Referring now to Fig. 1, a device 100 for bioimpedance measurement will be discussed. The device 100 is configured to be worn by the person, e.g. by comprising a carrier 102 which may be attached to or worn around a body part of the person. The carrier 102 may be implemented in form of a wearable, such as a wristband, or be supported by a wearable, such as a wristband for a watch, e.g. a smartwatch, or a bracelet.

However, it should be realized that the carrier 102 may be arranged in form of other wearables, such as any wearable, e.g. in the form of a band or a strap, suitable to be worn around other body parts, such as an ankle, an arm portion, a leg portion or a chest portion.

Also, it should be realized that the wristband 102 in Fig. 1 is not drawn to scale and that units which are arranged in or on the wristband 102 are exaggerated in size and shown outside the wristband 102 for illustrative purposes. Also, Fig. 1 shows an enlarged partial cross-section of the wirstband 102 to illustrate placement of electrodes 120 in the wristband 102.

The device 100 may comprise a bioimpedance measurement unit 110 and a plurality of electrodes, commonly referenced 120. The bioimpedance measurement unit 110 includes a bioimpedance measurement circuit 130 adapted to perform bioimpedance measurements on the person wearing the carrier 102 via a plurality of different groups 120-1, 120-2, ..., 120-n of the plurality of electrodes 120, wherein each group 120-1, 120-2, ..., 120-n comprises at least one pair of electrodes 120.

The bioimpedance measurement circuit 130 is configured to measure a respective impedance at each one of a plurality of portions of a body part of the person via a respective group of electrodes 120-1, ..., 120-n. The measurement of bioimpedance in a plurality of portions of a body part may be used for identifying the person.

Each group 120-1, ..., 120-n of electrodes may be referred to as an impedance measurement channel. The number n of groups or, correspondingly, impedance measurement channels is an implementation choice and may be at least one, at least two, at least three, at least four or at least five. Preferably, the number n of impedance measurement channels is at least 10, more preferably at least 20 and even more preferably at least 40.

The electrodes 120 are connected to the bioimpedance measurement circuit 130 via a switching circuit 132 configured to selectively connect groups of electrodes 120 to the bioimpedance measurement circuit 130. The bioimpedance measurement circuit 130 is configured to measure a bioimpedance via the electrode group to which the bioimpedance measurement circuit 130 is currently connected. The switching circuit 132 may be configured to control which impedance measurement channel should be used at each instant. The switching circuit 132 may include a set of switches, such as relays or transistor-based switches for selectively connecting input/output terminals of the bioimpedance measurement circuit 130 to the electrodes 120.

The functions and logic controlling the operation of the bioimpedance measurement circuit 130 and the switching circuit 132 may be implemented in a respective or in a common integrated circuit, an application specific integrated circuit (ASIC), a microcontroller unit (MCU) or a field-programmable gate array (FPGA).

In the illustrated device 100, each group of electrodes 120-1, 120-2, ..., 120-n includes four electrodes 120a-d. The electrode groups 120-1, ..., 120-n are shown to include distinct, non-overlapping, subsets of the electrodes 120. It is also possible to configure the electrode groups to have electrodes 120 in common such that the electrode groups overlap. As an example, a first electrode group may include a first through fourth electrode (e.g. 120a-120d in Fig. 1) and a second electrode group may include the second through fifth electrode (e.g. 120b-120e in Fig. 1). It is also possible to configure electrode groups with a smaller number of common electrodes such as only two or only one common electrodes. It should also be realized that electrodes within a group need not be formed by a sequence of adjacent electrodes. Rather, the electrodes of a group may be distributed along the wristband 102.

The electrodes 120 may be contact-less electrodes 120, adapted to only be capacitively coupled to the user. Each electrode 120 may include a separate conductive portion integrated inside the carrier 102. When the carrier 102 is worn on the person, the electrodes 120 will accordingly be separated from direct contact with the skin of the person by the electrodes 120 being integrated in the wristband 102 (which may be made from an electrically insulating material) and a part of the wristband 102 being arranged between the electrodes 120 and the skin of the person. The electrodes 120 may further be separated from the skin by an airgap between the carrier 102 and the skin of the person. Alternatively, the wristband 102 may have a shape (e.g. a non-uniform thickness along a circumference of the wrisband 102) such that the wristband 102 when worn on the wrist may place the electrodes 120 to be separated from the skin by an airgap. Thus, the electrodes 120 may only be capacitively coupled to the skin of the wrist.

Each conductive portion may include a conductive material such as stainless steel, copper, aluminum, gold, silver, silver-chloride or carbon, to name a few. The conductive material may be arranged as a thin layer which may be embedded in the carrier 102. A thin layer may for instance be glued to a surface of the carrier 102 or attached by mechanical attachment means, and then be covered by a capping layer which may form part of the carrier 102. According to further options a conductive portion may be formed by a patch of conductive ink deposited on the surface of the wristband 102, or by a conductive yarn incorporated into the surface of the wristband 102 (e.g. interwoven with a strap of a woven material), before being covered by a capping layer.

The carrier 102 may be formed of, or comprise at least between the electrodes 120 and a surface of the carrier 102 which is to face skin of the person when being worn, an electrically insulating material. The electrodes 120 being embedded in the carrier 102 ensures that there will be an insulating interface between the electrodes 120 and the carrier 102 such that the electrodes 120 are only capacitively coupled to the skin of the person.

It should be realized that instead of being embedded in the carrier 102, the electrodes 120 may be arranged on a surface of the carrier 102 which will not be facing the skin of the person when the carrier 102 is worn.

According to an embodiment, the carrier may include a flexible material, such as a woven material, a textile, a rubber or silicone material, which may facilitate the carrier being worn by the person. The strap may alternatively include a (more) rigid material. The strap may for example include leather or a plastic material.

The bioimpedance measurement circuit 130 may be adapted to perform a tetra-polar impedance measurement via the electrode groups 120-1, ..., 120-n. An impedance measurement will now be described in connection with the electrode group 120-1. An impedance measurement may be performed in a corresponding manner via the further measurement electrode groups 120-2, ..., 120-n. The bioimpedance measurement circuit 130 may be configured to measure an impedance via the electrode group 120-1 by providing a stimulation signal to the electrodes 120a and 120d of the group 120-1. The stimulation signal will by the capacitive coupling between the electrodes 120a and 120d and the skin of the person induce a varying voltage signal in the person. The bioimpedance measurement circuit 130 may be configured to measure the voltage induced in the person based on the capacitive coupling between the skin and the electrodes 120b and 120c. The measured voltage is indicative of bioimpedance of the person and may be used for determining the bioimpedance. It should also be realized that the tetra-polar impedance measurement may be performed using another relation between the electrodes used in the measurement. For instance, it is not necessary that the electrodes used for providing the stimulation signal are arranged on opposite sides of inner electrodes for acquiring the voltage measurement indicative of bioimpedance. On the contrary, according to another example, electrodes 120a and 120b may be used for providing a stimulation signal, whereas electrodes 120c and 120d may be used for measuring a voltage indicative of bioimpedance.

As an alternative to the tetra-polar impedance measurement configuration, the system 100 may include groups of electrodes 120-1, ..., 120-n including only two skin electrodes. The bioimpedance measurement circuit 130 may accordingly be configured to perform a bi-polar impedance measurement via electrode groups 120-1, ..., 120-n including only two electrodes. A bi-polar impedance measurement may be implemented in a manner corresponding to the above-described tetra-polar measurement however with the difference that in a measurement via an electrode group, the stimulation signal and the voltage measurement is performed via a same pair of electrodes.

The electrodes 120 may be distributed on a carrier, such as the carrier 102 in order to be arranged in different positions in relation to the person, when the carrier 102 is worn. This implies that bioimpedance measurements may be performed in a plurality of positions on the skin of the person.

The bioimpedance measurement unit 110 may further comprise a signal generator 134. The signal generator 134 may be configured to generate a stimulation signal, which may, via the switching circuit 132, be provided to electrodes 120 for, by capacitive coupling, inducing a varying voltage signal in the person.

The signal generator 134 may include a controlled current source configured to transmit an AC stimulation signal of a predetermined amplitude and frequency between, for example, the electrodes 120a and 120d. The amplitude of the stimulation signal may for example be in the range of 10 $\mu$A to 10 mA.

The signal generator may be configured to generate a stimulation signal of a specific frequency in order to allow measuring a bioimpedance based on the stimulation signal.

However, it should be realized that measuring bioimpedance for a plurality of frequencies may provide more detailed information of a characteristic of the person. For instance, in identification of a person using bioimpedance, it may be advantageous to make a plurality of bioimpedance measurements using a plurality of frequencies.

The stimulation signal may be generated to present a frequency in accordance with a current frequency channel selected from a set of frequency channels F. The set of frequency channels F may for instance cover, or cover a sub-range of, 100 kHz to 10 MHz. Each frequency channel f within the set of frequency channels F may be a single predetermined frequency channel or cover a relatively narrow frequency band.

The bioimpedance measurement circuit 130 may then be configured to, for each group of electrodes 120-1, ..., 120-n, measure an impedance $Z_{i,f}$ in each frequency channel f of a set of frequency channels F.

The signal generator 134 may be controlled for generating a sequence of frequency settings of the AC stimulation signals. These frequency settings may be controlled for controlling the measurements being performed using the device 100.

Since the electrodes 120 are capacitively coupled to the skin of the person, the AC signal in the skin of the person induced by the stimulating signal does not have a defined DC baseline in the person. Therefore, it may be desired to set a fixed operating point at the electrode terminals both for the stimulating signal and for acquiring the measurement signal. In this sense, it is advantageous to generate an AC stimulation current signal without a DC offset component of the current signal, such that an operating point may be accurately set.

Thus, the signal generator 134 may be combined with a DC eliminating unit 136, which may remove a DC offset component of the current signal so that the signal generator 134 in combination with the DC eliminating unit 136 may provide an output of an AC stimulation current signal without a DC offset component.

The bioimpedance measurement unit 110 may further be configured to control an operating point at the electrode terminals both for the stimulating signal and for acquiring the measurement signal.

The bioimpedance measurement unit 110 may thus comprise a bias network 138. The bias network 138 provides a fixed operating point. The bias network 138 may be connected, e.g. directly connected, or via a bias resistor, at electrode terminals for receiving the stimulating signal to induce a varying voltage in the skin of the person and at electrode terminals for acquiring the measurement signal. Thus, the bias network 138 may in this manner control the operating point of all electrode terminals.

By the bias network 138 being connected at electrode terminals for acquiring the measurement signal, the bias network 138 may be used for setting a fixed operating point at an input terminal of the bioimpedance measurement circuit 130. The use of the bias network 138 may thus facilitate correct processing of the received signal by the bioimpedance measurement circuit 130.

The device 100 may further comprise a distance detector 140. The distance detector 140 may be configured to determine a distance between the electrodes 120 and the skin of the person during bioimpedance measurements.

According to an embodiment, the distance detector 140 may be configured to detect a time of propagation of a signal or an attenuation of a signal. This may be used in an optical or electrical measurement, wherein an electromagnetic wave, such as a light wave or radio frequency wave, is transmitted and a reflected wave is detected. For instance, the distance detector 140 may include a light source configured to transmit a light pulse towards the skin and a light detector for detecting light being reflected by the skin.

The distance detector 140 may thus be arranged at a surface of the carrier 102 for enabling sending a signal towards the skin and receiving a reflected signal.

According to another embodiment, the distance detector 140 may be configured to detect an electrical property that may be dependent on the distance between the electrodes 120 and the skin. For instance, the distance detector 140 may comprise a circuit configured to detect an impedance caused by the distance between the electrodes 120 and the skin. Thus, the device 100 may comprise distance measurement electrodes for determining an impedance dependent on the distance between the bioimpedance measurement electrodes 120 and the skin.

According to an embodiment, the device 100 may comprise a plurality of distance detectors 140 at different positions of the carrier 102. This may allow correct determination of the bioimpedance, even if the carrier 102 is not arranged with a consistent distance to the skin for the entire carrier 102, as the plurality of distance detectors 140 may ensure that a distance that applies to a particular measurement may be determined. For instance, if the carrier 102 is worn as a strap around a body part, the carrier 102 may not have a single common distance to the skin throughout a circumference of the carrier 102.

The distance detector 140 may be configured to detect a distance simultaneously with bioimpedance measurement being performed. This may ensure that the distance between the electrodes 120 and the skin are known for the time when measurements are performed and that distance does not change between a point in time of determining the distance and a point in time of the performing the bioimpedance measurement.

It may be realized that distance detection may be performed at regular time intervals during bioimpedance measurement, such as a few times per minute.

However, if the distance detection may affect the result of the bioimpedance measurement, e.g. if an impedance measurement is performed also for determining the distance, the distance detection may not be performed simultaneously with bioimpedance measurement. In such case, the device 100 may be configured to perform distance detection immediately before or after bioimpedance measurement. If bioimpedance measurements are to be made during a relatively long period of time, such as when doing bioimpedance measurements for a plurality of different frequencies, the device 100 may be configured to alternate between distance detection and bioimpedance measurements.

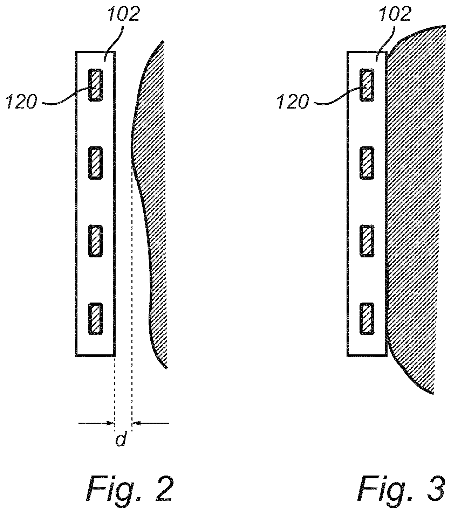

As mentioned above, the distance detector 140 may be arranged, at least in some embodiments, at a surface of the carrier 102, which means that the distance detector 140 may determine a distance d of an airgap between the carrier 102 and the skin, as illustrated in Fig. 2. This determined distance may be used together with a known arrangement of the electrodes 120 within the carrier 102, such that a total distance between the electrodes 120 and the skin, i.e. the distance from the electrode 120 to the surface of the carrier 102 + the distance from the surface of the carrier 102 to the skin, may be determined by adding a constant, known value to the determined distance.

It should also be realized that the distance detector 140 may not always be necessarily included in the device 100. If the carrier 102 is configured for being arranged in tight contact with the body part, it may be ensured that a surface of the carrier 102 is in contact with skin of the person. Then, the distance between the electrode 120 and the skin may be known and correspond to the distance between the electrode 120 and the surface of the carrier 102 as established in manufacturing of the device 100, as illustrated in Fig. 3.

The bioimpedance measurement circuit 130 may be configured to process a measurement signal received from the electrodes 120 being set by the switching circuit 132 to acquire the measurement signal. The bioimpedance measurement circuit 130 may also receive the stimulation signal.

The bioimpedance measurement circuit 130 may include an analog-to-digital converter for sampling and digitizing the resulting voltage induced in the person and detected by a selected pair of electrodes.

A relation between the measurement signal and the stimulation signal is an indication of bioimpedance of the person. However, the relation between the measurement signal and the stimulation signal is also dependent on the capacitive coupling between the electrodes and the skin, both for inducing a voltage in the person and for acquiring the measurement signal. Thus, there will be a combined impedance of the capacitive couplings between electrodes and the skin and the bioimpedance of the person.

The absolute value of the combined impedance may be estimated from the ratio between the amplitude of the measured voltage and the amplitude of the stimulation signal. A phase of the combined impedance may be estimated from a phase difference between the stimulation signal and the measured voltage. The bioimpedance measurement circuit 130 may thus calculate the absolute value of the combined impedance and the phase of the combined impedance in a digital domain.

Once the combined impedance is calculated, a contribution of the capacitive coupling may be removed so as to determine the bioimpedance. The contribution of the capacitive coupling may be determined based on the distance between the electrodes 120 and the skin. As mentioned above, the distance may be known by the detection made by the distance detector 140 and/or by a pre-determined distance based on the distance between the electrodes 120 and the surface of the carrier 102 facing or in contact with the person.

The bioimpedance measurement circuit 130 may be configured to estimate an impedance for the capacitive coupling between the electrodes 120 and the skin on the distance. The impedance may be estimated based on knowledge of the insulating material of the carrier 102 and on an assumption that a gap between the surface of the carrier 102 and the skin is filled by air.

The bioimpedance measurement circuit 130 may thus subtract the estimated impedances of the capacitive couplings between the electrodes 120 and the skin from the combined impedance to obtain a bioimpedance measurement result.

It should be realized that the bioimpedance measurement circuit 130 may process the received signals in other ways for determining a bioimpedance measurement result. For instance, the bioimpedance measurement circuit 130 may, based on the determined distance between the electrodes 120 and the skin, model the effect of the capacitive coupling on the stimulation signal and on the measurement signal so as to extract the signal being induced in the person and a corresponding measured voltage in the person, so as to obtain an estimate of the absolute value of the bioimpedance from the ratio between the amplitude of the measured voltage and the amplitude of the induced signal, and an estimate of the phase of the bioimpedance may be estimated from a phase difference between the induced signal and the measured voltage.

According to another embodiment, the device 100 may be used for acquiring calibration data for the person before starting making bioimpedance measurements. In calibration, impedance measurement data may be acquired for a number of different distances for generating calibration data. The calibration data may thus provide information of the measurement signal for the different distances.

The calibration data may then enable making good estimations of contribution of the capacitive coupling between the electrodes 120 and skin to the measurement signal, such that the calibration data may be used for estimating the impedance of the capacitive coupling when determining the bioimpedance. For instance, based on a determined distance, corresponding information of the impedance of the capacitive coupling may be fetched from the calibration data for use in determining the bioimpedance.

Alternatively, the calibration data may provide reference data sets for the measurement signal for different distances between the electrodes and the skin for a specific person. Each reference data set may provide characteristic information of an impedance measured for a specific person at a specific distance between the electrodes and the skin of the person. Hence, a combined impedance of the capacitive couplings and the bioimpedance may be characteristic for the person and may be represented by the reference data sets.

This implies that the bioimpedance measurement unit 130 need not necessarily separately determine the bioimpedance from the measurement signal. Rather, the bioimpedance measurement unit 130 may determine the combined impedance from the capacitive couplings and the bioimpedance. Then, the combined impedance may be directly compared to a corresponding reference data set, depending on the distance between the electrodes and the skin. The comparison may be used in order to determine whether the person for whom the measurement is performed corresponds to the person represented by the reference data set. Thus, a static physiological characteristic may be determined as a combined impedance for capacitive couplings between electrodes and skin and the bioimpedance.

The bioimpedance measurement circuit 130 may further comprise a filter for eliminating large changes in magnitude of the measurement signal. It should be realized that changes in distance between the electrodes and the skin may cause very large changes in magnitude of the measurement signal. Thus, a very large change in magnitude of the measurement signal cannot possibly be due to the varying voltage signal in the person and it can rather be deduced that the large change is due to a change in distance between the electrodes and the skin. The change in magnitude may prevent determining the bioimpedance for a time period in which the change is occurring. Thus, such a time period may be removed by the filter from further processing.

When a large change in magnitude of the measurement signal is detected, a bioimpedance measurement may be interrupted. Then, a (new) distance between the electrodes 120 and the skin may be detected by the distance detector 140 and a new bioimpedance measurement may be performed, wherein processing of the measurement signal takes the new distance into account.

As mentioned above, the functions and logic controlling the operation of the bioimpedance measurement circuit 130 may be implemented in an integrated circuit, an application specific integrated circuit (ASIC), a microcontroller unit (MCU) or a field-programmable gate array (FPGA). However, it should be realized that at least the processing in digital domain of the measurement results may be performed in any type of processing unit 150, such as a Central Processing Unit (CPU), which may execute a software program for performing the processing. This may be particularly useful for processing involving comparing measurement data with reference data, wherein the processing unit 150 may be able to access a memory 160 in which the reference data may be stored.

Thus, the device 100 may further comprise a processing unit 150, which may or may not perform (part of) the processing of the bioimpedance measurement circuit 130.

The processing unit 150 may further be configured to control functionality of all parts of the device 100. The processing unit 150 may be dedicated for the bioimpedance measurements performed by the device 100, but may alternatively be implemented as a processing unit 150 of a wearable, such as a smart watch, in which the device 100 is embedded.

The device 100 may thus comprise one or more processing units, such as a central processing unit (CPU), which may execute the instructions of one or more computer programs in order to implement functionality of the device 100. Thus, the device 100 may comprise a single processing unit, which may provide functionalities as separate threads within the processing unit.

The device 100 may alternatively comprise an implementation as firmware arranged e.g. in an embedded system, or as a specifically designed processing unit, such as an Application-Specific Integrated Circuit (ASIC), a microcontroller unit (MCU) or a Field-Programmable Gate Array (FPGA).

The device 100 may further comprise a memory 160, which may be configured to store each measured impedance in a storage structure in the form of a measurement array, forming a measurement data set. The memory 160 may include a volatile memory such as a volatile random access memory (RAM), or a non-volatile memory such as a flash-based memory. The label M will in the following be used to refer to both the measurement data set and the measurement array.

The memory 160 may further store a reference data set R in the form of a reference array including a storage position corresponding to each storage position of the measurement array. The label R will in the following be used to refer to both the reference data set and the reference array.

The reference data set R may be a reference data set providing characteristic information of bioimpedance of the person so as to enable identification of the person. The reference data set R may then be used in processing of measurement data, wherein an actual bioimpedance is determined based on the measurement data.

However, the memory 160 may store plurality of reference data sets, wherein each reference data set forms calibration data providing a combined impedance measurement for a specific distance between the electrodes 120 and the skin.

The device 100 may further comprise an identification unit, which may e.g. be implemented in the processing unit 150. The identification unit may be configured to receive impedance measurements from the bioimpedance measurement unit 110. The impedance measurements may be received as an actual bioimpedance of the person or as a combined impedance of the capacitive couplings between the electrodes and the skin and the bioimpedance. The received measurements may form a measurement data set, which may be processed by the identification unit in order to determine an identity of the person.

A measurement data set may enable identification of the person based on a characteristic of the person, such that the measurement data set may distinguish between different persons.

The identification unit may be configured to determine one or more features based on the received measurement data set. The one or more features may then be compared to reference value(s) which may each be associated with an identity of the person. The identification unit may alternatively be configured to compare the measurement data set with a corresponding reference data set in order to determine whether a difference between the measurement data set and the reference data set is sufficiently small so that the measurement data set is received from the person associated with the reference data set.

The measurement data set may be acquired to provide a metric associated with a physiological characteristic of the person. Thus, the measurement data set may comprise a plurality of measurements at different positions and/or different frequencies of impedance of the person.

In use of the device 100, the switching circuit 132 may connect the bioimpedance measurement circuit 130 to one electrode group at a time. An active impedance measurement channel may thus be changed in a "time-division manner" wherein the bioimpedance measurement circuit 130 may measure an impedance via only one electrode group at a time. For each impedance measurement channel, the bioimpedance measurement circuit 130 may measure an impedance in each frequency channel f of the total set of frequency channels F. The bioimpedance measurement circuit 130 may measure the resulting impedance for one frequency channel f at a time. The switching circuit 132 may thereafter cycle through all remaining impedance measurement channels such that an impedance is measured in each frequency channel f via each electrode group 120-1, ..., 120-n.

It should be realized that the bioimpedance measurement unit 110 may comprise a plurality of bioimpedance measurement circuits 130. If there are equal number of bioimpedance measurement circuits 130 and electrode groups 120-1, ..., 120-n, each electrode group may be connected to a dedicated bioimpedance measurement circuit 130. This implies that the bioimpedance measurements for several (all) impedance measurement channels may be performed simultaneously.

Now, an example of processing a measurement array M representing the measurement data set for determining whether the measurement array M corresponds to a reference array R will be described.

The measurement array M includes, for each group of electrodes 120-1, ..., 120-n, and for each frequency channel f, a storage position for storing a representation of a measured impedance. The measurement array may be in the form a one-dimensional data array or vector.

As an example, the measurement array M may have a structure of

$$[Z_{\text{group 1,f=ch\#1}}; Z_{\text{group 2,f=ch\#1}}; \ldots ; Z_{\text{group n,f=ch\#1}};$$

$$Z_{\text{group 1,f=ch\#2}}; Z_{\text{group 2,f=ch\#2}}; \ldots ; Z_{\text{group n,f=ch\#2}};$$

$$\ldots$$

$$Z_{\text{group 1,f=ch\#k}}; Z_{\text{group 2,f=ch\#k}}; \ldots ; Z_{\text{group n,f=ch\#k}}]$$

where each element denotes the impedance measurement via measurement electrode group i of the plurality of measurement electrodes 120, at frequency channel #j of the set of frequency channels F.

As an example, the reference array R may have a structure of

$$[R_{\text{group 1,f=ch\#1}}; R_{\text{group 2,f=ch\#1}}; \ldots ; R_{\text{group n,f=ch\#1}};$$

$$R_{\text{group 1,f=ch\#2}}; R_{\text{group 2,f=ch\#2}}; \ldots ; R_{\text{group n,f=ch\#2}};$$

$$\ldots$$

$$R_{\text{group 1,f=ch\#k}}; R_{\text{group 2,f=ch\#k}}; \ldots ; R_{\text{group n,f=ch\#k}}]$$

where each element denotes a reference impedance for the electrode group i of the plurality of measurement electrodes 120, at frequency channel #j of the set of frequency channels F.

The reference data set R may be a predetermined data set, established by performing bioimpedance measurements on a person with the device 100 one or more times and storing a representation of the measurements as the reference data set R. In case of plural measurements, an average of the measurements may be stored as the reference data set R.

The identification unit may be configured to compare the measurement data set M to the reference data set R.

The identification unit may be configured to determine a difference between the measurement array M and the reference array R. The identification unit may be configured to determine the difference by calculating a difference between a measured impedance and a reference impedance stored at each storage position of the measurement array M and the reference array R.

With reference to the above example structure of the measurement array M, the identification unit may accordingly be adapted to calculate:

$$[Z_{\text{group 1,f=ch\#1}} - R_{\text{group 1,f=ch\#1}}; Z_{\text{group 2,f=ch\#1}} - R_{\text{group 2,f=ch\#1}}; \ldots ]$$

Based on this calculation the identification unit may further be adapted to calculate a distance, e.g. in Euclidean sense, between the measurement array M and the reference array R. It should be noted that this represents only one possible way calculating a distance or degree of similarity between the measurement data set and the reference data set and that other calculations and norms also may be used.

Based on the processing of the measurement data set in relation to the reference data set, the identification unit may determine whether an identity of the person wearing the device 100 corresponds to the identity of the person for whom the reference data set was acquired.

A visual marker (e.g. a line, a dot or other graphical shape) may be provided on the carrier 102 to allow the person to wear the carrier 102 with a consistent rotation in relation to a body part through-out and between occasions of use. Especially, this may facilitate wearing the carrier 102 with a same orientation as when the reference data set R was established.

The identification unit may output data indicating a success or failure of authenticating the person (i.e. the person wearing the device 100 corresponds to a person of the reference data set or not). However, the memory 160 may also store several reference data sets for several persons, such that the identification unit may output data indicating an identity of the person or failure to identify the person at all.

The identification unit may be implemented in the processing unit 150. Alternatively, the identification unit may be implemented in a separate integrated circuit, ASIC, MCU or FPGA.

Further, the identification unit need not necessarily be provided within the device 100. On the contrary, the identification unit 144 may be arranged in a remote, external unit, such that acquired measurement data sets may be transmitted to the external unit for identifying of the person. The identification unit may be arranged in a dedicated external unit for authentication of persons, such that authentications of different persons using devices 100 may be performed in a common external unit.

The device 100 may further comprise a communication unit 170 for communication with external units. The communication unit 170 may be configured to receive information to be transmitted from the processing unit 150 and may be configured to forward information that is received from external units to the processing unit 150.

The communication unit 170 may include a wireless transceiver configured to communicate with an external unit. The wireless transceiver may be a Bluetooth or Bluetooth LE transceiver, an NFC transceiver, a WLAN transceiver or implement any other conventional type of wireless communications technology. The device 100 is however not limited to a wireless communication interface for communicating the output data. The communication unit 170 may alternatively provide communication to an external unit via a wired communication interface, such as a USB-interface.

The identification unit may be configured to generate data indicating a successful identification of the person in a manner trusted by the external unit. The identification unit may thus output data indicating a successful identification, which may be provided by the communication unit 170 directly to an external unit, requiring a user identification, or to an external unit which may use the output data to extract or generate information based on the successful identification, which information may in turn be used for identifying the user with respect to a service or device.

As mentioned above, the identification unit need not necessarily be arranged in the device 100. In such case, the communication unit 170 may be configured to communicate measurement data sets in order to allow identification of the person to be performed by an external unit.

The device 100 may further comprise a display 180, which may present information to a person wearing the device 100. The processing unit 150 may output information to the display 180 so as to, for instance, indicate a status of the device 100, such that a person may be notified when the device 100 is malfunctioning or when a battery of the device needs recharging. The processing unit 150 may also output results of identification of a person on the display 180 so that the person may be informed whether the device 100 successfully identifies the person.

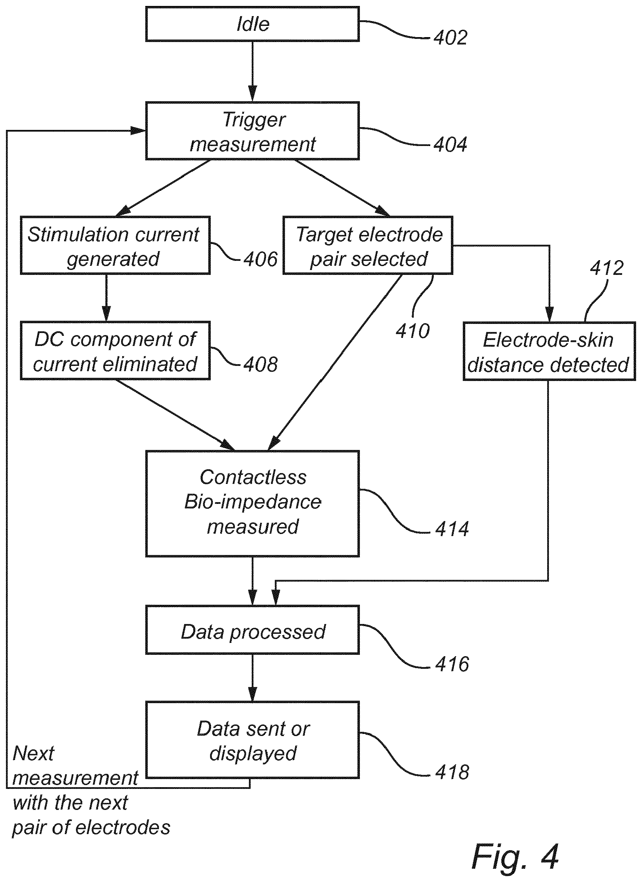

Referring now to Fig. 4, a process of performing bioimpedance measurements using the device 100 will be described.

The device 100 may be in an idle state 402, wherein the device 100 is ready to start bioimpedance measurements on a person. In the idle state, a trigger 404 for starting measurements may be provided. The trigger may be provided in any manner, e.g. based on input to the device 100 that bioimpedance measurement is to be made, whereby the processing unit 150 may trigger the bioimpedance measurement unit 110 to start measurements.

The bioimpedance measurement may include several simultaneous or parallel steps in order for a bioimpedance to be determined.

Firstly, the bioimpedance measurement may include generating 406 a stimulation current signal. The stimulation current signal may be generated by the signal generator 132. If the signal generator provides a DC component to the generated signal, the signal may also go through eliminating 408 of the DC component of the stimulation current signal.

Secondly, the bioimpedance measurement may include selecting 410 target electrode pair(s). The selecting of target electrode pair(s) may involve, for tetrapolar measurement, selecting both an electrode pair which is to receive the stimulation current signal and an electrode pair which is to sense a measurement signal indicative of the varying signal induced in the person.

Thirdly, the bioimpedance measurement may include detecting 412 a distance between the electrodes 120 and the skin. In a set-up as illustrated in Fig. 3, the detection of the distance may not be necessary, since the carrier 102 is arranged in contact with skin such that the distance between the electrodes 120 and the skin is given by the (known) distance between the electrodes 120 and the surface of the carrier 102.

The generated stimulation signal and a detected signal by the selected electrode pair may be provided to the bioimpedance measurement circuit 130. Thus, the bioimpedance measurement circuit 130 may perform measuring 414 of the bioimpedance. This measure is indicative of bioimpedance, but is also affected by the capacitive coupling between the electrodes 120 and the skin.

Then, processing 416 of the measured impedance may be performed, wherein the processing also has information of the distance between the electrodes 120 and the skin, either from the detection of the distance or based on the distance being known when the carrier 102 is arranged in contact with skin.

The processing may take the distance into account in order to determine a static physiological characteristic, as described above, either by actual determination of the bioimpedance or by using the combined impedance of the capacitive couplings and the bioimpedance together with information of the distance between the electrodes and the skin for which the combined impedance was acquired.

The determined data, after processing, may be sent 418 to a further unit for further handling of the determined data. For instance, the data may be stored in the measurement array M for enabling later processing for identification of the person. Alternatively, the data may be displayed, e.g. for simple display of a determined bioimpedance based on the processing of data.

Then, the process may return to step 404 for triggering a measurement with a next group of electrodes 120.

In the above the inventive concept has mainly been described with reference to a limited number of examples. However, as is readily appreciated by a person skilled in the art, other examples than the ones disclosed above are equally possible within the scope of the inventive concept, as defined by the appended claims.

Although the device 100 is described above as comprising a plurality of groups of electrodes 120, it should be realized that the device 100 may be provided with only a single group of electrodes 120 (such as one pair of electrodes for bipolar measurement or two pairs of electrodes for tetrapolar measurement) and that there may be applications wherein it would be sufficient to determine a bioimpedance in a single position of a body part of the person using the device 100.

**Claims**

1.  A device for bioimpedance measurement, said device (100) comprising:

    a carrier (102), which is configured to be worn by a person,
    a plurality of electrodes (120), which are arranged at a distance from a surface of the carrier (102) facing the person when the carrier (102) is worn, the plurality of electrodes (120) being configured for forming a capacitive coupling to the person when the carrier (102) is worn,
    a bioimpedance measurement unit (110), which is supported by the carrier (102) and which is configured to, via at least one pair of electrodes among said plurality of electrodes (120), provide a stimulation signal and acquire a measurement signal indicative of bioimpedance of the person, wherein the bioimpedance measurement unit (110) is configured to take a distance between the at least one pair of electrodes and skin of the person into account in order to estimate a static physiological characteristic of a bioimpedance.

2.  The device according to claim 1, further comprising a distance detector (140), which is supported by the carrier (102), and which is configured to determine a distance between the at least one pair of electrodes and the skin of the person.

3.  The device according to claim 2, wherein the distance detector (140) is configured to detect a distance based on an optical measurement or an electrical measurement.

4.  The device according to any one of the preceding claims, wherein the bioimpedance measurement unit (110) is configured to estimate a contribution to a measured signal from an insulating interface formed by the distance between the electrodes (120) and the skin of the person in order to determine an absolute value and/or phase of an bioimpedance.

5.  The device according to any one of the preceding claims, wherein the bioimpedance measurement unit (110) comprises a bioimpedance measurement circuit (130), which is configured to perform bioimpedance measurements on the person via a plurality of different groups among said plurality of electrodes (120).

6.  The device according to any one of the preceding claims, wherein the bioimpedance measurement unit (110) comprises a bias network (138), which is configured to provide an operating voltage at an input terminal of the bioimpedance measurement circuit (130).

7.  The device according to any one of the preceding claims, wherein the bioimpedance measurement unit (110) comprises a signal generator (134; 136), which is configured to generate an AC stimulation current signal without a DC offset component of the current signal.

8.  The device according to any one of the preceding claims, wherein the bioimpedance measurement unit (110) is configured to filter the measurement signal in time domain to eliminate large changes in magnitude of the measurement signal.

9.  The device according to any one of the preceding claims, further comprising a memory (160) storing calibration data, wherein the bioimpedance measurement unit (110) is configured to process the measurement signal using the calibration data.

10. The device according to claim 9, wherein the calibration data comprises bioimpedance measurement results for different distances between the at least one pair of electrodes and the skin of the person.

11. The device according to any one of the preceding claims, wherein the carrier (102) is in the form of a wristband.

**12.** The device according to any one of the preceding claims, wherein the bioimpedance measurement unit (110) is configured to determine a measurement data set based on measurement signals acquired via a plurality of different groups of electrodes and based on each frequency channel of a set of frequency channels of the stimulation signal, wherein the device (100) further comprises an identification unit, which is configured to process the measurement data set in relation to at least one reference data set for identifying the person.

**13.** A method for bioimpedance measurement, said method comprising:

> providing (406) a stimulation signal;
> acquiring (414) a measurement signal, wherein the providing of the stimulation signal and the receiving of the measurement signal is performed via at least one pair of electrodes (120) which are capacitively coupled to the person;
> accessing information of a distance between the at least one pair of electrodes (120) and skin of the person; and
> processing (416) the measurement signal in view of the distance between the at least one pair of electrodes (120) and the skin of the person in order to estimate a static physiological characteristic of a bioimpedance.

**14.** The method according to claim 13, wherein said accessing of information of the distance comprises measuring (412) the distance between the at least one pair of electrodes (120) and the skin of the person using a distance detector (140).

*Fig. 1*

EP 3 539 468 A1

Fig. 2

Fig. 3

Fig. 4

Europäisches
Patentamt

European
Patent Office

Office européen
des brevets

# EUROPEAN SEARCH REPORT

Application Number

EP 19 16 1966

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/057506 A1 (LUNA MICHAEL EDWARD SMITH [US] ET AL) 26 February 2015 (2015-02-26) * abstract; figures 1-8 * * paragraphs [0018] - [0056] * | 1-14 | INV. A61B5/053 A61B5/00 |
| A | US 2016/165450 A1 (HUNT ALEXANDER [SE] ET AL) 9 June 2016 (2016-06-09) * abstract; figures 1-10 * * paragraphs [0002] - [0018], [0033] - [0085] * | 1-14 | |
| A | US 2006/052678 A1 (DRINAN DARREL D [US] ET AL) 9 March 2006 (2006-03-09) * abstract; figures 1-9 * * paragraphs [0015] - [0094] * | 1-14 | |
| A | WO 2016/149829 A1 (GESTURELOGIC INC [CA]) 29 September 2016 (2016-09-29) * abstract; figures 1-7 * * paragraphs [0005] - [0018], [0047] - [0180] * | 1-14 | |

TECHNICAL FIELDS
SEARCHED (IPC)

A61B

The present search report has been drawn up for all claims

| Place of search | Date of completion of the search | Examiner |
|---|---|---|
| The Hague | 25 June 2019 | Carta, Riccardo |

EPO FORM 1503 03.82 (P04C01)

## ANNEX TO THE EUROPEAN SEARCH REPORT
## ON EUROPEAN PATENT APPLICATION NO.

EP 19 16 1966

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2019

| Patent document cited in search report | | Publication date | Patent family member(s) | | Publication date |
|---|---|---|---|---|---|
| US 2015057506 | A1 | 26-02-2015 | AU | 2013323116 A1 | 16-04-2015 |
| | | | AU | 2013323117 A1 | 23-04-2015 |
| | | | AU | 2013323118 A1 | 16-04-2015 |
| | | | CA | 2886651 A1 | 03-04-2014 |
| | | | CA | 2887142 A1 | 03-04-2014 |
| | | | CA | 2887393 A1 | 03-04-2014 |
| | | | CN | 203252647 U | 30-10-2013 |
| | | | EP | 2900127 A2 | 05-08-2015 |
| | | | EP | 2900129 A2 | 05-08-2015 |
| | | | EP | 2900136 A1 | 05-08-2015 |
| | | | US | 2014094675 A1 | 03-04-2014 |
| | | | US | 2015057506 A1 | 26-02-2015 |
| | | | US | 2015216475 A1 | 06-08-2015 |
| | | | US | 2015230756 A1 | 20-08-2015 |
| | | | WO | 2014052986 A2 | 03-04-2014 |
| | | | WO | 2014052987 A1 | 03-04-2014 |
| | | | WO | 2014052988 A2 | 03-04-2014 |
| US 2016165450 | A1 | 09-06-2016 | CN | 107004078 A | 01-08-2017 |
| | | | EP | 3227814 A1 | 11-10-2017 |
| | | | US | 2016165450 A1 | 09-06-2016 |
| | | | WO | 2016089445 A1 | 09-06-2016 |
| US 2006052678 | A1 | 09-03-2006 | US | 2006052678 A1 | 09-03-2006 |
| | | | US | 2006058593 A1 | 16-03-2006 |
| | | | US | 2010268111 A1 | 21-10-2010 |
| | | | WO | 2006029034 A2 | 16-03-2006 |
| | | | WO | 2006029035 A1 | 16-03-2006 |
| WO 2016149829 | A1 | 29-09-2016 | NONE | | |

EPO FORM P0459

For more details about this annex : see Official Journal of the European Patent Office, No. 12/82